# EUROPEAN PATENT SPECIFICATION

(11) **EP 1 264 603 B1**
(45) Date of publication and mention of the grant of the patent: **23.12.2009**
(21) Application number: 01114110.8
(22) Date of filing: 10.06.2001
(51) Int. Cl.: A61K 49/00, A61K 49/04, A61K 51/04

(54) **Use of L-polynucleotides for in vivo imaging**
Verwendung von L-Polynukleotiden zur diagnostischen Bilderzeugung
Utilisation de L-polynucleotides pour l'imagerie diagnostique

(43) Date of publication of application: 11.12.2002
(73) Proprietor: Noxxon Pharma AG, 13355 Berlin (DE)
(72) Inventor: Klussmann, Sven, 10709 Berlin (DE); Wlotzka, Britta, Dr., 12203 Berlin (DE)
(74) Representative: Bohmann, Armin K.

(56) References cited:
- WO-A-94/20523
- WO-A-96/34879
- WO-A-98/08856
- WILLIAMS KELLY P ET AL: "Bioactive and nuclease-resistant L-DNA ligand of vasopressin." PROCEEDINGS OF THE NATIONAL ACADEMY OF SCIENCES OF THE UNITED STATES, vol. 94, no. 21, 1997, pages 11285-11290, XP002185340 1997 ISSN: 0027-8424
- NOLTE A ET AL: "MIRROR-DESIGN OF L-OLIGONUCLEOTIDE LIGANDS BINDING TO L-ARGININE" NATURE BIOTECHNOLOGY, NATURE PUBLISHING, US, vol. 14, 1 September 1996 (1996-09-01), pages 1116-1119, XP002039162 ISSN: 1087-0156
- DOLLE FREDERIC ET AL: "A general method for labeling oligodeoxynucleotides with 18F for in vivo PET imaging." JOURNAL OF LABELLED COMPOUNDS AND RADIOPHARMACEUTICALS, vol. 39, no. 4, 1997, pages 319-330, XP001038207 ISSN: 0362-4803
- MOYROUD E ET AL: "Synthesis and Enzymatic Digestion of an RNA Nonamer in Both Enantiomeric Forms" TETRAHEDRON, ELSEVIER SCIENCE PUBLISHERS, AMSTERDAM, NL, vol. 56, no. 11, March 2000 (2000-03), pages 1475-1484, XP004203025 ISSN: 0040-4020

## Description

The present invention is related to an L-polynucleotide for use in in vivo imaging, and the use of a kit comprising an L-polynucleotide.

Novel imaging methods based on radionuclides or other reporter systems in combination with specific targeting to a compound or structure or receptor system or a metabolic characteristic that is a unique marker for an indication like cancer, offer intriguing opportunities for the development of a new generation of diagnostic methods. These imaging methods will facilitate both the diagnosis and staging of a disease and enable the early assessment of the effectiveness of a therapy by providing a more accurate in vivo characterization of the disease status.

The different imaging methods and more particularly in vivo imaging methods known to date can visualize entities of tissues ranging from structure, physiology, metabolism, drug distribution, molecular pathways and receptor distribution. The sensitivity of the different methods ranges from the detection of millimolar concentrations in magnetic resonance imaging to picomolar concentrations in positron emission tomography. The specificity is ranging from the detection of tissue density and water content to a clearly identified molecule linked to a radiolabel. The investigation of biochemical pathways, ligand/receptor interaction or biodistribution of a molecule of interest and simultaneously avoiding a potential pharmacologic effect in tracer studies requires a method with high sensitivity.

One type of in vivo imaging method is magnetic resonance imaging referred to herein also as MRI. MRI is based on the absorption and emission of energy in the radio frequency range of the electromagnetic spectrum. Images are produced based on spatial variations in the phase and frequency of the radio frequency energy being absorbed and emitted by the imaged object. With different methods images of the body can be generated in 2D and 3D. Magnetic resonance imaging is currently used for producing anatomical images but can also give information on the physico-chemical state of tissues, flow diffusion and motion information.

A typical reporter system is a complex of a paramagnetic metal ion such as gadolinium. The paramagnetic field creates many oscillating fields as it tumbles through a water environment. Most paramagnetic metal ions are toxic. To alleviate this effect these metal ions are complexed with other molecules to prevent complex formation in the body. MRI can be used for angiography, the imaging of blood in the arteries and veins of the body and for the detection of changes in size or shape of tissues and organs in oncology.

More particularly, for imaging the brain MRI's multiplanar image capabilities, as well as its ability to discriminate between tissues, makes it extremely sensitive to pathological processes. MRI is the most accurate imaging modality for demonstrating metastatic disease in the bone marrow. This method is used in the determination of congenital and acquired disease of the thoracic aorta and will be further developed for applications in the evaluation of the heart. In MRI angiography the blood flow in the arteries and veins of the body is imaged. MRI is the method of choice for the study of the joints and soft tissue. The objective of future developments will be target specific imaging and the determination of physiologic abnormality such as tumors, inflammatory diseases and neurodegenerative diseases.

MRI as such is described, e.g., in Brown M.A., Smelka R.C. and Semelka R.C.; MRI: Basic Principles and Applications; Wiley-Liss, 2nd edition 1999; ISBN 0471330620.

A further in vivo imaging method is positron emission tomography referred to herein also as PET. PET as such is described, e.g., in Haberkorn U. and Bellemann M. E.; Positronen-Emissions-Tomographie; MMP 5 (20): 117 (1997). Compared to other imaging methods the advantage of PET imaging is its sensitivity for the detection of low concentrations of molecules. PET is advantageous in so far as it is more sensitive by a factor of one million than other techniques used to study regional metabolism and neuroreceptor activity in the brain and other body tissues. Since the nanomolar range is the concentration range of most receptor proteins in the body, positron emission tomography is ideal for this type of imaging. As imaging agents and more particularly as labels positron emitting isotopes are used such as ¹¹C, ¹³N, ¹⁵O and ¹⁸F all of them having short half lives. These radionuclides decay by positron emission. The emitted positron collides with a free electron usually within a distance of less than 1 mm from the point of emission. The interaction of the two subatomic particles results in a conversion of matter to energy in the form of two gamma rays. These high-energy gamma rays emerge from the collision point in opposite directions, and are detected by an array of detectors which surround the patient. The distribution of the positron emitting tracer is calculated by tomographic reconstruction procedures. Images in 2D and 3D can be generated. The major clinical applications of PET have been in cancer detection of the brain, breast, heart, lung and colorectal tumors. Another application is the evaluation of coronary artery disease by imaging the metabolism of heart muscle. Metabolic imaging of tumors by PET depends on accelerated metabolism in tumor versus normal tissue as increased glucose utilization, DNA synthesis and protein synthesis. Positron labelled sugars, amino acids and nucleotides have been used for tumor imaging corresponding roughly to the rate of growth of these tumors. Radionuclides such as ¹¹C and ¹⁸F can be used. Advances have been made in the use of ^{94m}Tc and ¹²⁴J for PET studies. Disease staging and assessment of therapeutic mechanism and efficacy are emerging not only for neurologic indications but also in cardiology and oncology.

A third method for in vivo imaging is single photon emission computed tomography referred to herein also as SPECT. In SPECT the position and distribution of a radionuclide is monitored. More particularly, the radiation is recorded by a gamma camera rotating around the patient for taking pictures from many angles. The data are converted by a computer to form a tomographic (cross-sectional) image. The calculation process is similar to that in X-ray computed tomography and in positron emission computed tomography. Because the emission sources, the injected radiopharmaceutical, are inside the body cavity, this task is far more difficult than for x-ray computer tomography (CT), where the source position and strength (outside the body) are known at all times. ^{99m}Tc is a commonly used isotope for this method. SPECT imaging is inferior to PET because of the resolution and sensitivity that can be attained due to the emitted energy of the different radionuclides used for both methods. However, there is considerably lower cost for instrumentation and less exposure of patients to radioactivity are associated with SPECT which makes the method attractive for clinical applications. SPECT as such is described, among others, in Bull U., Kirsch C.M. and Roedler H.D.; Single photon emission computed tomography (SPECT). Principles, results, outlook; ROFO Fortschr Geb Rontgenstr Nuklearmed 1983; 138 (4): 391; O'Connor M.K.; Instrument- and computer-related problems and artefacts in nuclear medicine; Semin Nucl Med 1996; 26(4): 256; and Mansoor M.R. and Heller G.V.; Gated SPECT imaging; Semin Nucl Med 1999; 29 (3): 271.

A prerequisite of all of these in vivo imaging methods is to have a label which may be correlated with a certain structure, compound, process, condition or disease. Such a label may be part of a compound which allows the targeting in the broadest sense of a compound, structure, process, condition or disease of interest. This kind of label bearing compound is referred to herein also as targeting tool, imaging agent or contrast agent. Depending, among others, on the particular in vivo imaging method to be used or applied some of the more prominent targeting tools are the followings.

### Monoclonal antibodies.

Due to the large molecular weight antibodies are cleared very slowly from the circulation. Their access to certain biological compartments can be very slow or factually be not existing at all. The slow clearance may necessitate waiting for days before a significant signal can be detected over the background of an excess of unbound ligand. These characteristics are limiting the usefulness of imaging technology in diagnosing acute conditions and precludes the use of short-lived radionuclides. Antibodies have been investigated as imaging agents in the diagnosis of inflammation and cancer. Several monoclonal antibodies reactive with antigens expressed on granulocytes, such as CD15, CD66 and CD67, have been developed. At least four anti-granulocyte antibodies have been tested for infection imaging. Each of these anti-granulocyte antibodies labelled with ^{99m}Tc allowed accurate delineation of infection and inflammation. A ¹²³I-labelled anti-VCAM-1 antibody showed good results in the evaluation of colonic inflammation in an animal model. A HMFG1-antibody directed against a mucin molecule which is expressed in a range of epithelial neoplasms such as breast, ovarian and non-small-cell lung cancer, was tested in patients with breast cancer. The use of antibodies is described, among others, in Wilson C.B. et al.; Quantitative measurement of monoclonal antibody distribution and blood flow using positron emission tomography and ¹²⁴Iodine in patients with breast cancer; Int J Cancer 1991; 47: 344; and Sans M. et al; ¹²³Iodine-labelled anti-VCAM-1 antibody scintigraphy in the assessment of experimental colitis; Eur J Gastroenterol Hepatol. 2001 Jan;13(1):31.

### Proteins.

The imaging characteristics of receptor-specific small proteins like cytokines and chemokines have been investigated for infection and inflammation. The specificity of uptake has been shown for many of these proteins. Unfortunately, all agents also accumulate to a certain extent in several non-target tissues. In particular, during the first hour after injection high background uptake is found. Early visualization of infection or inflammation foci in tissues with high background uptake is a problem and should be optimised. Toxicity is still the major drawback of using receptor-specific small proteins. The tested proteins are mediators of the inflammatory response and most of them elicit a specific response after binding to the receptor. The induction of biological activity is undesirable for scintigraphic imaging. In the ideal case the imaging agent binds to its receptor with high affinity without induction of any biological effects. ^{99m}Tc is the radionuclide of choice due to its cost-effectiveness, availability and desirable physical characteristics. The use of proteins for in vivo imaging is described, among others, in Van der Laken C.J. et al; Scintigraphic detection of infection and inflammation: new developments with special emphasis on receptor interaction; Eur J Nucl Med. 1998 May;25(5):535; and Boerman O.C. et al; Radiopharmaceuticals for scintigraphic imaging of infection and inflammation; Inflamm Res. 2001 Feb;50(2):55.

### Peptides.

Receptor-binding peptides labelled with γ-emitters like ¹²³J, ¹¹¹In or ^{99m}Tc are investigated for the non-invasive visualization of tissues. This technique is referred to as peptide-receptor radionuclide imaging, PRRI. The same peptides labelled with β-emitters such as ⁹⁰Y or ¹⁸⁸Rh can be used for the eradication of the receptor-expressing tissues referred to as peptide-receptor radionuclide therapy (PRRT). The use of peptides for in vivo imaging is described, among others, in Boerman O.C., Oyen W.J.G. and Corstens F.H.M.; Radio-Labeled Receptor-Binding Peptides: A new class of radiopharmaceuticals; Semin Nucl Med. 2000 Jul;30(3):195

One major problem associated with the use of radiolabeled peptides is nephrotoxicity caused by the relatively high retention of these compounds in the kidneys. The investigation of the mechanism revealed the adherence of peptides to the luminal membrane of the tubular cell of the kidney by electrostatic interaction. Positively charged peptides bind the negatively charged receptors on the tubular cell membrane.

It has been demonstrated that peptides are suitable tools for rapid imaging of infection and inflammation but that the undesired biological side effects of them seem to impede further clinical development.

### Antisense oligonucleotides.

The antisense approach to in vivo imaging has a number of disadvantages. The predominant mechanism of antisense chemotherapy is RNase H mediated degradation of the targeted mRNA resulting in mRNA destruction to achieve inhibition as described, e.g. in US 5,809,602. Subsequently the antisense oligonucleotide is released either to repeat the process or to exit the cell along with the covalently linked reporter group leading to the reduction of the number of mRNA targets or loss of the localization of the target specific signal. Another issue of concern is the steady-state copy number of mRNA per cell because successful antisense imaging will be dependent upon the presence of a minimum number of target mRNA per cell. Antisense chemotherapy as well as antisense imaging suffers from poor cellular uptake. The use of antisense oligonucleotides in in vivo imaging is described, among others in Hnatowich D.J.; Antisense Imaging: Where are we now?; Cancer Biother Radiopharm. 2000 Oct;15(5):447-57

### Aptamers.

Aptamers can be designed as high affinity and high specificity ligands for any protein or peptide target of interest. Aptamers are of a much lower molecular size than antibodies. Due to their physico-chemical properties they are very soluble and show fast clearance from the circulation after iv or sc administration. During synthesis aptamers can be easily modified for the subsequent introduction of a reporter group. The main disadvantage of aptamers is their limited in vivo stability. A number of different nucleases are abundant in body fluids and tissues leading to fast degradation and loss of the binding properties. In a feasibility study investigating an aptamer binding to human neutrophil elastase for inflammation imaging low background and a high signal to background ration could be observed (Charlton, J.; Sennello, J. and Smith D.: In vivo imaging of inflammation using an aptamer inhibitor of human neutrophil elastase. Chem&Biol, 4 (1997): 809 - 816). Apart from aptamers, i.e. target binding oligonucleotides, also non-specific oligonucleotides were used for in vivo imaging as described in Tavitian et al. (Tavition B. et al., Nature Medicine, Vol 4, April 1998, p. 467-471).

In principle, in vivo imaging techniques may be applied for the diagnosis and staging of many diseases and conditions. To further illustrate the potential of these techniques their application in diagnosis and staging of cancer and inflammation is illustrated in the following.

Tumors can be recognized by the overexpression of various receptor types. For example, somatostatin receptors are expressed in the majority of neuroendocrine tumors and in neuroblastomas, some medullary thyroid carcinomas, prostate cancers and small-cell lung cancers. In healthy tissue somatostatin receptors are expressed in the brain, gut, neuroendocrine, most lymphatic tissues, kidney, prostate and thyroid tissue. As a consequence, most tumors derived from these tissues express somatostatin receptors. Somatostatin is a peptide hormone of 14 amino acids and one disulfide bond. The wild type somatostatin shows fast metabolic degradation and is of limited use for in vivo applications. An analog of 8 amino acids named octreotide with improved metabolic stability was developed. An ¹¹¹In label linked to the octreotide and the diagnostic accuracy to visualize tumor lesions has been determined in large series of oncology patients. It was concluded that imaging parameters such as the peptide dose administered, the duration of the acquisition and the use of single photon emission computed tomography affected the sensitivity of somatostatin-receptor imaging. With this method good results were also achieved in the majority of patients with breast carcinoma and lymphoma. VIP, a 28-amino acid peptide with various biological activities, was also investigated for the use for scintigraphic imaging of tumor lesions. VIP receptors are widely expressed throughout the gastrointestinal tract and on various other cell types. VIP labelled with ¹²³I showed good performance in patients with colorectal cancer in terms of high sensitivity and high specificity. The application of this imaging agent is limited by the tendency of accumulation in the lungs and its laborious preparation procedure requiring HPLC purification. In addition the agent caused a transient drop in blood pressure. In prostate carcinomas gastrin-releasing peptide receptors are expressed and neurotensin receptors are found in Ewing's sarcomas, meningiomas and exocrine pancreatic tumors. The majority of breast cancers express calcitonin receptors.

A method for the early detection of acute or subacute infections or inflammatory states would allow earlier therapy and help to prevent complications leading to prolonged hospitalisation and delayed rehabilitation of the afflicted patients. In a number of inflammatory diseases such a method would enable the monitoring and assessment of the patients response ot the treatment resulting in the efficient therapy in indications such as rheumatiod arthritis, systemic vasculitidies, ulcerative colitis and Crohn's disease (Pike M.C.; Journal of Nuclear Medicine; Vol. 32, No. 11; 2034 - 2036 and citations therein). So far no method is available for the detection of intra-abdominal sources of inflammation and infection. The ideal radiopharmaceutical for inflammation imaging should accumulate rapidly in inflammatory/infectious foci and rapidly clear from noninflamed tissues. Due to the permeability of blood vessel in inflamed regions a certain degree of leakage into the interstitial space is taking place. A low molecular weight compound with good solubility is expected to be advantageous for these approaches. Receptors expressed preferentially on infiltrating leukocytes would be the ideal target candidates. Some techniques have been described for the detection of inflammatory sites. The most relevant are Gallium-67 scanning, the use of Indium-111 or ^{99m}Tc labeled leukocytes and ^{99m}Tc or ¹¹¹In-labeled agents such as anti-granulocyte antibodies and polyclonal immunoglobulins. The main disadvantage of the application of Gallium-67-citrate is the long time period required between injection and imaging. The leukocyte labelling with ¹¹¹In has considerable risk for the detection of false positive results due to gastrointestinal bleeding or swallowed leukocytes and the requirement of high radiation doses. The drawback of ^{99m}Tc-colloid labelled leukocytes were the in vivo oxidation of the technetium and the activation of the leukocytes resulting from the phagocytic process. Blood has to be obtained from the patient and leukocyte separation must be performed prior to labelling requiring skilled technical personnel. With the application of prelabeled agents such as monoclonal antibodies or chemotactic peptides allowing targeting of inflammation specific epitopes on the leukocyte membrane the imaging procedure is considerably facilitated. Ideal candidates for inflammation specific imaging would be agents for localization of neutrophils or monocytes by binding to cell surface receptors without activation of these cells preventing untoward side effects. It would be desirable to diagnose particular types of inflammatory responses like granulomatous versus neutrophilic versus eosinophilic on the basis of the specificity of the imaging agent applied.

International patent application WO 94/20523 is related to tumor targeting with L-enantiomeric oligonucleotide conjugates of immunoreagents and of chelated radtonucitnsS.

"Williams K.P.et al. (PNAS,vol.94,no.21,1997,pages 11285-11290) disclose bioactive and nuclease-resistant L-DNA ligand of vasopressin"

"Nolte A et al.(Nature Biotechnology,vol.14,01.Sept.1996,pages 1116-1119) is related to mirror-design of L-oligonucleotide ligands binding to L-arginine"

The problem underlying the present invention was to provide a targeting tool for the practice of in vivo imaging methods which is small in size, has a high affinity to the target if needed and a good solubility allowing for rapid clearance from the blood and other non-target tissues.

Another problem underlying the present invention was to provide for a targeting agent which is highly specific for a compound, structure such as a tissue or organ, a process or a disease and does not affect the biological activity of such compound, structure, process or disease.

According to the present invention this problem is solved by an L-polynucleotide for use in in vivo imaging whereby the L-polynucleotide is a Spiegelmer, the Spiegelmer shows binding activity towards a target or a part thereof and the target is a protein, peptide, small molecule or a pharmaceutically active compound and its metabolite.

In a further preferred embodiment the L-polynucleotide comprises an in vivo imaging label.

In another embodiment the in vivo imaging is a method selected from the group comprising magnetic resonance imaging, positron emission tomography, single photon emission computed tomography and optical imaging.

In a further preferred embodiment the label is selected from the group comprising paramagnetic metal ions, ⁷⁵Br, ¹¹C, ¹³C, ¹⁸F, ⁶⁷Ga, ¹¹¹In, ¹²⁰J, ¹²³J, ¹²⁴J, ¹²⁵J, ¹³¹J, ¹³N, ¹⁵O, ¹⁸⁶Re, ¹⁸⁸Re, ^{94m}Tc, ^{99m}Tc, ¹²⁹Xe gas, ⁸⁶Y and ⁹⁰Y.

In another embodiment the L-polynucleotide is a L-polydesoxyribonucleotide.

In an alternative embodiment the L-polynucleotide is a L-polyribonucleotide.

In a preferred embodiment the in vivo imaging is used for diagnosis, monitoring, assessment and/or staging of a disease or a condition and/or of a patient's response to a medical treatment.

In a more preferred embodiment the disease or condition is selected from the group comprising cancer, multiple sclerosis, rheumatoid arthritis, stroke, myocardial infarction, neurodegenerative diseases and inflammation.

In an even more preferred embodiment the cancer cells have an overexpression of at least one receptor type.

In a particularly preferred embodiment the L-polynucleotide is specific for a target.

In a further embodiment the target is a key target of the disease or condition.

In another embodiment the L-polynucleotide has a minimum length whereby the minimum length is selected from the group comprising 10, 15, 20, 25, 30, 35, 40, 45, 50, 55, 60, 70, 80, 90 and 100 nucleotides.

In a preferred embodiment the in vivo imaging is for the imaging of an organ.

In a further preferred embodiment the L-polynucleotide is specific for an organ.

In another preferred embodiment the L-polynucleotide is specific for a key compound of the organ.

In a further preferred embodiment the L-polynucleotide is a L-RNA and the in vivo imaging is for imaging of the kidney.

In an alternative preferred embodiment the L-polynucleotide is a L-DNA and the in vivo imaging is for the imaging of plasma, heart, lungs and/or liver.

In a further embodiment the in vivo imaging is in vivo optical imaging.

In a further embodiment the in vivo imaging is a magnetic resonance imaging and the L-polynucleotide is used as targeting tool.

In a further embodiment the in vivo imaging wherein the method is positron emission tomography and the L-polynucleotide is used as targeting tool.

In a further embodiment the in vivo imaging is single photon emission computed tomography and the L-polynucleotide is used as a targeting tool.

In a further embodiment the in vivo imaging is an in vivo optical imaging process wherein the L-polynucleotide is used as a targeting tool.

In an second aspect the invention is related to the use of kit comprising a L-polynucleotide for in vivo imaging, wherein the L-polynucleotide is a Spiegelmer, whereby the Spiegelmer shows binding activity towards a target or a part thereof and the target is a protein, peptide, small molecule or a pharmaceutically active compound and its metabolites.

The present invention is based on the surprising finding that it is possible to use L-polynucleotides being Spiegelmers, i.e. mirror image oligonucleotides, as targeting tools in factually all of the in vivo imaging methods known in the art. The Spiegelmers fit exactly to the profile of the targeting tools by being, if deemed necessary, very specific to a certain target and being small in size and exhibiting good solubility allowing for rapid clearance from the blood and other non-target tissues.

The term in vivo imaging is besides the general understanding as known in the art, to be particularly understood such as to designate the visualization of anatomical structures like the vascular system, organs or other tissues in the living body without major intervention for the purpose of detecting changes of shape, size and position of these anatomical structures by introducing imaging agents in body cavities or injection in blood vessels leading to a change in the contrast of the cavities or vessels. However, in vivo imaging may also comprise the visualization of molecular and cellular processes.

In addition, it is crucial to realize that due to the chemical nature of the L-polynucleotides these are not metabolised so that the pharmacokinetics underlying the use of L-nucleotides according to the present invention is not affected by any degradation processes which may otherwise be subject to changes depending on both the individual characteristics of the person subject to the in vivo imaging such as body weight etc., and the compound, structure, process or disease to be analysed using in vivo imaging procedures. In view of this increased stability of the L-polynucleotides the lifetime of the targeting tool is thus factually unlimited. Besides this more pharmacokinetic aspect also the aspect that no degradation products are derived from the L-polynucleotide as such is to be acknowledged as due to this no side effects will arise from the use of L-nucleotides in a living body. Such living body is preferably a mammal which is selected from the group comprising humans, monkeys, dogs, cats, horses, rats and mice. This unique feature of L-nucleotides resides in the fact that in a biological system there are no activities such as enzymes which could degrade the L-oligonucleotide as any nuclease activity is directed to the natural enantiomers, i. e. the D-enantiomers. Of particular importance is the fact the L-polynucleotides are not nephrotoxic.

Another tremendous advantage of using an L-polynucleotide, more particularly a Spiegelmer as described in the following, for in vivo imaging is that its binding region upon the target molecule or target structure can more or less be freely chosen. As a consequence such a target binding L-polynucleotide may bind at a site different from the site where other, typically naturally occurring ligands such as peptides or proteins bind. Because of this there won't be any triggering of a biological response which would otherwise arise from the binding of the naturally occurring ligand or an analogue thereof used for in vivo imaging purposes if the target is linked to a signalling cascade. Thus no unwanted side effect of the in vivo imaging method using an L-oligonucleotide do result.

The L-nucleotides may comprise further moieties. Such moieties are typically related to the labelling of the L-polynucleotides to allow its detection in in vivo imaging methods. Other additional moieties may be used for carrier purposes, modulation of pharmacokinetic behaviour and modulation of the physico-chemical properties and may be selected from the group comprising lipophilic moieties, peptides, proteins, carbohydrates and liposomes. It is to be understood that the L-oligonucleotide as defined herein may also comprise some D-oligonucleotides.

As used herein the term L-nucleotides or L-polynucleotides shall mean any nucleic acid which is composed of L-nucleotides, preferably composed of L-nucleotides only. About the length of such L-polynucleotides it is to be noted that it may range from one L-nucleotide to any number. However, lengths of at least 10, 15, 20, 25, 30, 35, 40, 45, 50, 55, 60, 70, 80, 90 and 100 nucleotides are preferred. The L-polynucleotides as used herein may thus also be L-oligonucleotides. Such oligonucleotides may be comprised of L-A, L-G, L-C, L-U, L-T and combinations thereof whereby L-A means the L-enantiomer of adenine etc.

It is within the present invention that the L-polynucleotides may be present as DNA or RNA, with both forms possibly being present as either double- or single-stranded. Typically, the L-polynucleotide is present as single-stranded nucleic acid which may form - defined - secondary structures due to the primary sequence and thus also tertiary structures. In such secondary structures there are also double-stranded stretches present in a variety of the L-polynucleotides which may be used for in vivo imaging according to the present invention. The L-polynucleotides, however, may also be present as double-stranded in the meaning that two strands which are complementary to each other, are hybridised. This may result in stabilization of the nucleic acids. The L-polynucleotide(s) may also be modified. This modification may be related to the individual nucleotide of the polynucleotide. Examples for such type of modification may be taken from.

As indicated above, the L-polynucleotide as used for in vivo imaging may be a so-called Spiegelmer.

Spiegelmers are nucleic acids comprising a number of L-nucleotides which show binding activities towards a target or a part thereof. The basic method of Spiegelmer generation is subject to the international patent application WO 98/08856Basically, this method relies on the so-called SELEX technique as described, e. g. in US 5,475,096 or EP 0 533 838. The method uses combinatorial DNA or RNA libraries comprising a randomised stretch of about 10 to about 100 nucleotides which are flanked by two primer binding regions at the 5' and 3' end. The generation of such combinatorial libraries is, for example, described in Konrad C., R. C., Giver, L., Tian, Y. and Ellington, A. D., 1996, Methods Enzymol., vol. 267, 336 - 367. Such a chemically synthesized single-stranded DNA library may be transferred into a double-stranded library via polymerase chain reaction. Such a library may already be used for selection purpose. The selection occurs such that the, typically single-stranded, library is contacted with a target molecule and the binding elements of the library are then amplified.

By repeating these steps several times oligonucleotide molecules may be generated having a significant binding activity towards the target used.

Spiegelmers, as said above, are actually L-polynucleotides which are generated such that D-polynucleotides are selected against a target molecule which is present in its non-naturally occurring enantiomer, and the nucleic acid binding thereto is then synthesized using L-nucleotides creating the L-polynucleotide, which is the Spiegelmer. This L-polynucleotide is capable of binding to the target molecule in its naturally occurring form. In case the target is a protein or peptide the non-naturally occurring enantiomer is the D-protein/peptide and the naturally occurring enantiomer is the L-protein/peptide.

Targets against which the Spiegelmers can actually be generated are viruses, proteins, peptides, nucleic acids, small molecules like metabolites of the metabolic pathways, pharmaceutically active compounds and their metabolites.

In connection with the present invention this specificity of the Spiegelmers may actually be used to target a certain structure, compound, process, condition or disease. This may be done such that the structure targeted or aimed at in connection with the in vivo imaging process comprises a chemical compound which is used as the target in the Spiegelmer generation process. As the in vivo imaging may also target a certain process condition or disease, an appropriate approach to the use of Spiegelmers in in vivo imaging methods aiming at these, would be to generate Spiegelmers against a chemical compound being involved in said process, condition or disease. It is acknowledged that this particular chemical compound shall be more or less characteristic for said process, condition or disease. It is within the skills of the man of the art to identify the compound which is characteristic of the structure, process, disease or condition to be monitored or displayed using in vivo imaging methods against which the Spiegelmer is directed. An especially interesting field of application for Spiegelmers would be the visualization of inflammatory processes using adhesion molecule binding Spiegelmers. Conditions like multiple sclerosis, rheumatoid arthritis, myocardial infarction, stroke, inflammatory bowel disease and a number of other pathological changes involving the inflammation cascade could be diagnosed

As used herein, a L-polynucleotide is specific for a target, if its binding characteristics are such that the binding affinity towards the particular target is increased compared to the binding characteristic of a non-target. Such non-target is typically a compound which was not used in the selection process as described in connection with the generation of Spiegelmers. However, it is to be noted that due to structural similarities a certain cross reactivity of the target specific Spiegelmer with non-targets may be observed similar to cross-reactivity of antibodies.

With regard to the physics underlying the in vivo imaging methods described herein, different in vivo imaging labels may be used. Factually said in vivo imaging label may be any label which is suitable to generate a signal which is detectable in any of the in vivo imaging methods. Typically, the following isotopes are used in connection with
PET: ¹¹C,¹⁸F, 15O, ^{94m}Tc, ¹²⁰J, ¹²⁴J, ⁸⁶Y, ¹³N, ⁷⁵Br;
SPECT : ^{99m}Tc, ¹²³J, ¹³¹J, ¹²⁵J, ⁶⁷Ga, ¹¹¹In, ¹⁸⁶Re, ¹⁸⁸Re, ⁹⁰Y
MRI: ¹³C, Gd, ¹²⁹Xe gas.

It is to be understood that the various isotopes used in connection with these in vivo imaging methods may be substituted by isotopes emitting a higher energy radiation. Due to this high energy radiation the L-polynucleotides may actually be used for therapeutic purposes specifically delivering the radiation to the targeted compound or structure which may by a tumor or the like. This change from the use of L-polynucleotides as contrast agent, targeting tool or imaging agent to therapeutic agent or a radiopharmaceutical is within the skills of the man of the art. The general principle on how to generate radiopharmaceuticals is described, e.g., in Boerman O.C., Oyen W.J.G. and Corstens F.H.M.; Radio-Labeled Receptor-Binding Peptides: A new class of radiopharmaceuticals; Semin Nucl Med. 2000 Jul;30(3):195.

For reason of illustration it is referred to receptor-binding peptides labelled with gamma emitters (Iodine-123, Indium-111, Technetium-99m) which could facilitate the visualization of receptor-expressing tissues noninvasively, a technique referred to as peptide-receptor radionuclide imaging (PRRI). In addition, labelled with beta-emitters (1-131, Yttrium-90, Rhenium-188, Re-186) these peptides also have the potential to eradicate receptor-expressing tissues: an approach referred to as peptide-receptor radionuclide therapy (PRRT). This labelling technique can also be applied to L-polynucleotides and the thus labelled L-polynucleotide then be used according to the present invention.

Besides the in vivo imaging methods already described above there is a further in vivo imaging method in which an L-polynucleotide and more particularly a Spiegelmer may be used as a targeting agent which is in vivo optical imaging (Weissleder, R., nature biotechnology, 19, April 2001, p. 316 - 317; Becker A. et al., nature biotechnology, 19, April 2001, p. 327- 331). The basic principle underlying this in vivo imaging method is the use of light photons, i.e. the use of near-infrared light (NIR) to stimulate NIR fluorochromes. Hemoglobin and water, the major absorbers of visible and infrared light, respectively, have their lowest absorption coefficient in the NR region around 650 - 900nm. Light photons can be used to measure different native parameters of tissue through which they travel. For example, absorption, scattering, polarization, spectral characteristics, and fluorescence. Recent developments are related to in vivo optical imaging result from the availability of targeted NIR fluorochromes, activable NIR fluorochromes, red-shifted fluorescent proteins and bioluminescent probes.

It is known in the art (Becker, supra; Weissleder, supra) that peptides may be labelled using such NIR labels which are used to image receptors using reflectance imaging. The present inventors have now discovered that L-oligonucleotides may be used for in vivo optical imaging. Thus optical in vivo imaging will be an alternative to radiochemical diagnosis. In a preferred embodiment the optical in vivo imaging using an L-polynucleotide is near-infrared fluorescence.

In a preferred embodiment NIR labels may be used in connection with L-polynucleotides and more particularly with Spiegelmers. The labelling of L-polynucleotides with appropriate labels and more particularly with NIR labels may be done using standard chemistry known to the skilled in the art.

Suitable labels may be fluorescent dyes like cyanine derivatives such as indodicarbocyanine (IDCC), indotricarbocyanine (ITCC); Cy5 and derivatives thereof. IDCC and ITCC are schematically represented as (1), whereby R represents the L-oligonucleotide and n = 2 for IDCC and n = 3 for ITCC.

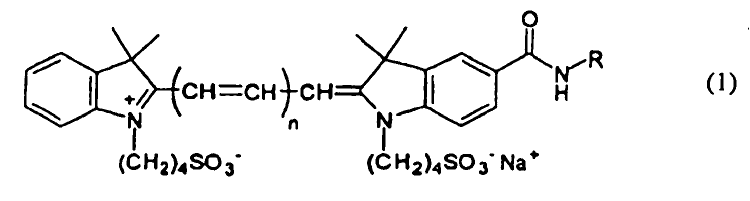

The labels may be excited when being in the body to be imaged, by the use of a pulsed, solidstate laser system using, e.g., an excitation wavelength of 740 nm; fluorescence may be detected using, e.g. an intensified charge-coupled device (CCD) camera. Long-wave pass filters were used to cut off reflected excitation light and wavelengths between 780 and 900 nm are detected. (For a more detailed description see Becker et al. supra.)

An example for an application of in vivo optical imaging using an L-polynucleotide is cancer imaging, more particularly the use for tumor detection; imaging of specific enzymes in intact tumor environments using smart optical probes; in vivo imaging of angiogenesis; in vivo imaging of gene expression; imaging of transcriptional activation, post-transcriptional modulation and specific protein-protein interactions, imaging of receptors at various stages of cancer such as prostate and breast cancer. Accordingly, as the other in vivo imaging methods also in vivo optical imaging may be used for receptor targeting and internalisation into the cell.

Although all of these aforementioned applications of in vivo optical imaging may also be realized using different in vivo imaging methods such as MRI, PET and SPECT, the use of in vivo optical imaging is in so far advantageous as it is applicable at the cellular and molecular level.

It is to be understood that basically the in vivo imaging methods using L-polynucleotides may be used for any purpose for which said methods are known to be applied. These purposes comprise, among others, diagnosis, monitoring, assessment and/or staging. Subjects of these activities may either be a diseased or healthy individual, preferably a mammal such as a human being. In addition, the particular in vivo imaging methods may also be applied to diagnosing, monitoring, assessing or staging an individual, preferably a patient and more particularly the patient's response to a treatment such as a medical treatment.

In case the in vivo imaging is applied in connection with cancer use may be made from receptors which are typically overexpressed in tumor tissue. Accordingly, a L-nucleotide, preferably a Spiegelmer, could be used being specific for the particular receptor. Further indications comprise inflammation, multiple sclerosis, rheumatoid arthritis, stroke, myocardial infarction. Cancers and tumors which may be targeted comprise, among others, breast cancer prostate cancer, kidney cancer, sarcoma, lymphoma, liver cancer, brain cancer, colon cancer, pancreas cancer, bladder cancer, lung cancer, endometriosis, thyroid cancer and the like.

The in vivo imaging methods using an L-polynucleotide may also be used for the display of a tissue or an organ. Such display may occur in connection with the above specified other application aspects of the in vivo imaging methods. Due to the differences in pharmacokinetics and metabolism between L-DNA and L-RNA already some forms of application can be realized. For example, initial concentrations of L-oligonucleotides are typically dependent on blood flow for both L-DNA and L-RNA.

It is to be noted that any of the in vivo imaging methods described herein, namely MRI, PET SPECT and in vivo optical imaging, are known as such in the art. However, the use of L-oligonucleotides being Spiegelmers in such methods has been developed by the present inventors. Except the difference in the targeting tools the practice of said in vivo imaging methods remain unchanged compared to the practice using targeting tools as known in the art such as monoclonal antibodies, peptides and proteins.

Generally, the incorporation of the in vivo imaging label may be performed by standard techniques.

Many US patents, such as, for example, US Patent No. 4,707,440, deal with modified polymers, which contain a detectable chemical group. The polymers can be polynucleotides and oligonucleotides, but they are neither stabilized against a degradation by naturally occurring nucleases nor selected by a special process, so that they bind specifically with high binding affinity to target structures. Special embodiments of these detectable molecules are named in US Patents No. 4,843,122 and 4,943,523. An individual nucleotide, modified in this way, is claimed in US Patent No. 4,952,685. The use of these agents in imaging processes is disclosed in US Patent No. 4,849,208. From these documents of the prior art the technical teaching how to incorporate a chemical group either acting as or comprising a label for in vivo imaging or acting as a means to incorporate into or bind such a label to the targeting tool may be taken. Said means may be in some cases a complexing agent, particularly when it comes to the use of metal ions as labels such as in MRI and SPECT.

Due to the toxicity of many of the radionuclides used for different imaging methods complexing agents, such as polydentate, open-chain or cyclic complexing ligands with O, S and N donor atoms are used for tight binding. Examples are polyaminopolycarboxylic acids reduced by a hydrogen, a hydroxy group and/or an acetic acid group like ethylenediaminetetraacetic acid. Suitable complexing agents are described, e.g., in EP 0 485 045, EP 0 071 564 and EP 0 588 229. The complex can contain all paramagnetic metal ions used in MRI diagnosis or radioisotopes used in scintigrafic methods.

These complexes or the chemical group either acting as or comprising a label for in vivo imaging or acting as a means to incorporate into or bind such a label to the targeting tool shall be referred to herein also as the reporter group. The linkage of the L-polynucleotide and Spiegelmer, respectively, with the reporter group can take place not only by the 5'-OH group of the sugar of the terminal nucleotide, but also by other functional groups, which can be generated from the 5'-OH group, such as, e.g., an amino, carboxy or thiol group. Such nucleotides carrying amino or carboxy groups are known and can be produced easily, e.g. via a phosphoramidite derivative. The synthesis of a 5'-deoxy-5'-amino-uridine is described in J. Med. Chem. 22, 1273 (1979) as well as in Chem. Lett. 6, 601 (1976). 4'-Carboxy-5'-deoxy-uridine is accessible as described in J. Med. Chem. 21, 1141 (1978), or Nucleic Acids Symp. Ser. 9, 95 (1981). These methods can easily be transferred to L-nucleotides and Spiegelmers.

The reporter group can also be introduced subsequent to an amino, carboxy or thiol modifications to the 3'- end of a Spiegelmer. The nucleobases offer an especially great variety for linking the reporter group with the nucleotides. A linkage by the amino groups in 2-position in the purines and in 4-position in the pyrimidines can take place directly. But it is often more advantageous first to modify the purines or pyrimidines and to link these derivatized bases with the reporter groups. Suitable derivatized nucleobases are described, e.g., in Biochemie 71, 319 (1989), Nucl. Acids Res. 16, 4937 (1988) or Nucleosides Nucleotides 10, 633 (1991).

The reporter group can be introduced to the 3'-end of a Spiegelmer as well by using an accordingly modified solid support. The nucleobases offer a great variety for linking the reporter group with the nucleotides. A linkage by the amino groups in 2-position in the purines and in 4-position in the pyrimidines can take place directly. It can be more advantageous first to modify the purines or pyrimidines and to link these derivatized bases with the complexing agents.

In addition to that, the inventors have perceived new methods on how to introduce a radioactive isotope of halogens into Spiegelmers. This labelling method is based on the efficient conjugation reaction between the oligonucleotides bearing a 3'-phosphorothioate monoester group with a radio[halogeno]benzyl-bromoacedamid as described in example 1.

Also the inventors have perceived a new method for the labelling of L-ribonucleic acids (L-polyribonucleotides) as described in example 3

The kit which is to be used in accordance with the present invention may be manufactured for the purpose of in vivo imaging. Said kit may comprise the L-polynucleotide for use in accordance with the present invention. The diverse forms of L-polynucleotides contained in such kit are those described herein in connection with the use of L-polynucleotides for in vivo imaging. Accordingly, the L-polynucleotides are Spiegelmers and preferably already contain the in vivo imaging label as specified herein. However, it may also be possible that the kit comprises only the ingredients, i. e. the non-labelled Spiegelmer and the label so that the labelled L-oligonucleotide or Spiegelmer may generated by the user of the kit reacting the starting materials. This may be recommendable due to the short half lives of the radioisotopes or in case the labelling is done via a linkage which may not be stable for the whole of the shelf life of the kit which may go clearly beyond the residence time of the L-oligonucleotide in the organism subject to the in vivo imaging. Also, said kit may further comprise some instructions on how to use the kit, buffers, stabilizers and the like.

The invention is further illustrated by the following examples.

### Example 1

### Synthesis of N-(4-fluorobenzyl)-2-bromoacetamide.

¹⁸F labelled N-(4-fluorobenzyl)-2-bromoacetamide may be produced according to the following reaction as taught by to Dolle F. et al (Dolle F. et al.; Journal of Labelled Compounds and Radiopharmaceuticals Vol. XXXIX, No. 4, p. 319 - 330). 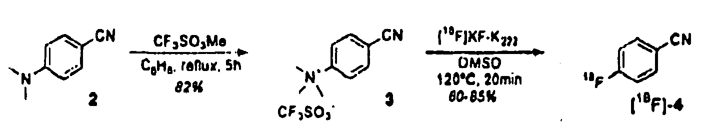 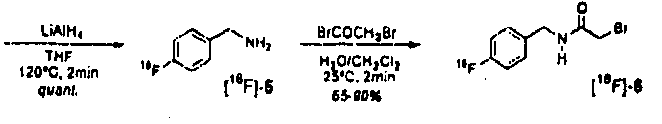 This compound may be used to incorporate basically any halogen such as F or I to a nucleic acid, preferably L-polynucleotides such as Spiegelmers. F and I are suitable labels or reporter groups for in vivo imaging.

### Example 2

### Labelling of deoxypolynucleotides

Starting from, e.g., N-(4-[¹⁸F] fluorobenzyl)-2-bromoacetamide or N-(4-[¹²⁵I]iodobenzyl)-2-bromoacetamide the label, i.e. ¹⁸F or ¹²⁵ I, may be introduced into a deoxypolynucleotide according to the following reaction which is taught by Dolle et al. (supra). 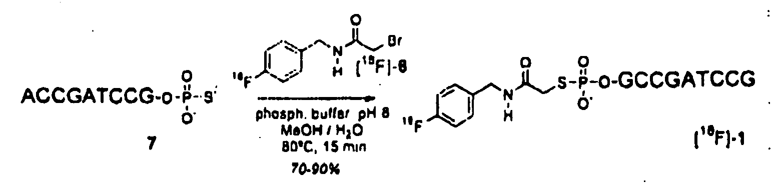 The nucleotide sequence shown is actually only for illustrative purposes. The method as described above is not limited to any particular sequence and may be either applied die L-deoxypolynucleotides or to D- deoxypolynucleotides.

### Example 3

### Labelling ofpolyribonucleotides

Starting from, e.g., N-(4-[¹⁸F] fluorobenzyl)-2-bromoacetamide or N-(4-[¹²⁵ I]iodobenzyl)-2-bromoacetamide the label, i.e. ¹⁸F or ¹²⁵ I, may be in principle introduced into a polyribonucleotide according to the reaction taught by Dolle et al. (supra) and displayed in example 2 herein. However, according to the method taught by Dolle et al. (supra), the radiolabel is introduced at the 3'- end of the deoxypolyribonucleotide such as a DNA - Spiegelmer. In case a polyribonucleotide is to be labelled using this method it is necessary to avoid a nucleophilic attack of the 2'-O of the 2'- OH group to the phosphate of the thiophosphate group. The introduction of an additional deoxy-L-nucleotide or a different suitable 2'- modification such as a 2'-amine or 2'alkyl (such as, e.g.,methyl, ethyl, propyl) to the sequence is a straight forward and successful approach for the labelling of RNA polynucleotides, i.e. polyribonucleotides. It is to be noted that this procedures can preferably be applied to L-polyribonucleotides such as RNA-Spiegelmers irrespective of the particular sequence of the L-polyribonucleotide. However, it may also be applied to D-polyribonucleotides.

The thiophosphate at the 3'-position of the deoxyuridine can be transformed in high yield with [¹⁸F]N-(4-fluorobenzyl)-2-bromoacetamid to the radiolabeled Spiegelmer. In so far the present invention refers in a further aspect to L-polynucleotide comprising both a L- RNA moiety and a 3' terminal deoxyribonucleotide. Preferably, the RNA moiety is a Spiegelmer. In a further preferred embodiment, the L-polynucleotide consists entirely of L-nucleotides.

## Claims

1. An L-polynucleotide for use in in vivo imaging, wherein the L-polynucleotide is a Spiegelmer, whereby the Spiegelmer shows binding activity towards a target or a part thereof and the target is a protein, peptide, small molecule or a pharmaceutically active compound and its metabolite.

2. The L-polynucleotide according to claim 1, wherein the L-polynucleotide comprises an in vivo imaging label.

3. The L-polynucleotide according to claim 1 or 2, wherein the in vivo imaging is a method selected from the group comprising magnetic resonance imaging, positron emission tomography, single photon emission computed tomography and optical imaging.

4. The L-polynucleotide according to claim 2 or 3, wherein the label is selected from the group comprising paramagnetic metal ions, ⁷⁵Br, ¹¹C, ¹³C, ¹⁸F, ⁶⁷Ga, ¹¹¹In, ¹²⁰I, ¹²³I, ¹²⁴I, ¹²⁵I, ¹³¹I, ¹³N, ¹⁵N, ¹⁸⁶Re, ¹⁸⁸Re, ^{94m}Tc, ^{99m}Tc, ¹²⁹Xe gas, ⁸⁶Y and ⁹⁰Y.

5. The L-polynucleotide according to any of claims 1 to 4, wherein the L-polynucleotide is a L-polydesoxyribonucleotide.

6. The L-polynucleotide according to any of claims 1 to 5, wherein the L-polynucleotide is a L-polyribonucleotide.

7. The L-polynucleotide according to any of claims 1 to 6, wherein the in vivo imaging is used for diagnosis, monitoring, assessment and/or staging of a disease or a condition and/or of a patient's response to a medical treatment.

8. The L-polynucleotide according to claim 7, wherein the disease or condition is selected from the group comprising cancer, multiple sclerosis, rheumatoid arthritis, stroke, myocardial infarction, neurodegenerative diseases and inflammation.

9. The L-polynucleotide according to claim 8, wherein the cancer cells have an overexpression of at least one receptor type.

10. The L-polynucleotide according to any of claims 1 to 9, wherein the L-polynucleotide is specific for a target.

11. The L-polynucleotide according to claim 10, wherein the target is a key target of the disease or condition.

12. The L-polynucleotide according to any of claims 1 to 11, wherein the L-polynucleotide has a minimum length whereby the minimum length is selected from the group comprising 10, 15, 20, 25, 30, 35, 40, 45, 50, 55, 60, 70, 80, 90 and 100 nucleotides.

13. The L-polynucleotide according to any of claims 1 to 12, wherein the in vivo imaging is for the imaging of an organ.

14. The L-polynucleotide according to claim 13, wherein the L-polynucleotide is specific for an organ.

15. The L-polynucleotide according to claim 14, wherein the L-polynucleotide is specific for a key compound of the organ.

16. The L-polynucleotide according to any of claims 1 to 15, wherein the L-polynucleotide is a L-RNA and the in vivo imaging is for imaging of the kidney.

17. The L-polynucleotide according to any of claims 1 to 16, wherein the L-polynucleotide is a L-DNA and the in vivo imaging is for the imaging of plasma, heart, lungs and/or liver.

18. Use of a kit comprising a L-polynucleotide for in vivo imaging, wherein the L-polynucleotide is a Spiegelmer, whereby the Spiegelmer shows binding activity towards a target or a part thereof and the target is a protein, peptide, small molecule or a pharmaceutically active compound and its metabolite

## Patentansprüche

1. L-Polynukleotid zur Verwendung bei einem *in vivo*-bildgegebenen Verfahren, wobei das L-Polynukleotid ein Spiegelmer ist, wobei das Spiegelmer Bindungsaktivität bezüglich eines Zielmoleküls oder eines Teiles davon aufweist und das Zielmolekül ein Protein, Peptid, kleines Molekül oder eine pharmazeutisch aktive Verbindung und sein Metabolit ist.

2. L-Polynukleotid nach Anspruch 1, wobei das L-Polynukleotid eine *in vivo-*bildgebende Markierung umfasst.

3. L-Polynukleotid nach Anspruch 1 oder 2, wobei das bildgebende Verfahren ein Verfahren ist, das ausgewählt ist aus der Gruppe umfassend Magnet-Resonanz-Darstellung, Positron-Emissionstomographie, Einzelphotonenemissionscomputertomographie und optische Bildgebung.

4. L-Polynukleotid nach Anspruch 2 oder 3, wobei die Markierung ausgewählt ist aus der Gruppe umfassend paramagnetische Ionen, ⁷⁵Br, ¹¹C, ¹³C, ¹⁸F, ⁶⁷Ga, ¹¹¹In, ¹²⁰I, ¹²³I, ¹²⁴I, ¹²⁵I, ¹³¹I, ¹³N, ¹⁵O, ¹⁸⁶Re, ¹⁸⁸Re, ^{94m}Tc, ^{99m}Tc, ¹²⁹Xe-Gas, ⁸⁶Y und ⁹⁰Y.

5. L-Polynukleotid nach einem der Ansprüche 1 bis 4, wobei das L-Polynukleotid ein L-Polydesoxyribonukleotid ist.

6. L-Polynukleotid nach einem der Ansprüche 1 bis 5, wobei das L-Pclynukleotid ein L-Polyribonukleotid ist.

7. L-Polynukleotid nach einem der Ansprüche 1 bis 6, wobei das *in vivo*-bildgebende Verfahren verwendet wird für die Diagnose, Überwachung, Bewertung, und/oder das Staging einer Erkrankung oder eines Zustandes und/oder der Reaktion eines Patienten auf eine medizinische Behandlung.

8. L-Polynukleotid nach Anspruch 7, wobei die Erkrankung oder der Zustand ausgewählt ist aus der Gruppe umfassend Krebs, multiple Sklerose, rheumatoide Arthritis, Schlaganfall, Myokardinfarzierung, neurodegenerative Erkrankungen und Entzündung.

9. L-Polynukleotid nach Anspruch 8, wobei die Krebszellen eine Überexpression von wenigstens einem Rezeptortyp zeigen.

10. L-Polynukleotid nach einem der Ansprüche 1 bis 9, wobei das L-Polynukleotid spezifisch für ein Zielmolekül ist.

11. L-Polynukleotid nach Anspruch 10, wobei das Zielmolekül ein Schlüssel-Zielmolekül der Erkrankung oder des Zustandes ist.

12. L-Polynukleotid nach einem der Ansprüche 1 bis 11, wobei das L-Polynukleotid eine Minimallänge aufweist, wobei die Minimallänge ausgewählt ist aus der Gruppe umfassend 10, 15, 20, 25, 30, 35, 40, 45, 50, 55, 60, 70, 80, 90 und 100 Nukleotide.

13. L-Polynukleotid nach einem der Ansprüche 1 bis 12, wobei das in vivo-bildgebende Verfahren der bildgebenden Darstellung eines Organs dient.

14. L-Polynukleotid nach Anspruch 13, wobei das L-Polynukleotid spezifisch für ein Organ ist.

15. L-Polynukleotid nach Anspruch 14, wobei das L-Polynukleotid spezifisch für eine Schlüsselverbindung des Organs ist.

16. L-Polynukleotid nach einem der Ansprüche 1 bis 15, wobei das L-Polynukleotid eine L-RNA ist und das in vivo-bildgebende Verfahren der bildgebenden Darstellung der Niere dient.

17. L-Polynukleotid nach einem der Ansprüche 1 bis 16, wobei das L-Polynukleotid eine L-DNA ist und das *in vivo*-bildgebende Verfahren der Darstellung von Plasma, Herz, Lungen und/oder Leber dient.

18. Verwendung eines Kits umfassend ein L-Polynukleotid für ein *in vivo*-bildgebendes Verfahren, wobei das L-Polynukleotid ein Spiegelmer ist, wobei das Spiegelmer Bindungsaktivität bezüglich eines Zielmoleküles oder eines Teiles davon zeigt und das Zielmolekül ein Protein, Peptid, kleines Molekül oder eine pharmazeutisch aktive Verbindung und sein Metabolit ist.

## Revendications

1. Poly-L-nucléotide pour une utilisation dans une imagerie in vivo, dans lequel le poly-L-nucléotide est un Spiegelmer, dans lequel le Spiegelmer manifeste une activité de liaison à l'égard d'une cible ou d'une partie de celle-ci, et la cible est une protéine, un peptide, une petite molécule, ou un composé pharmaceutiquement actif et son métabolite.

2. Le poly-L-nueléotide selon la revendication 1, dans lequel le poly-L-nucléotide comprend un marqueur d'imagerie in vivo.

3. Le poly-L-nucléotide selon la revendication 1 ou 2, dans lequel l'imagerie in vivo est un procédé choisi parmi le groupe comprenant l'imagerie par résonance magnétique, la tomographie par émission de positons, la tomographie par émission de photon simple assistée par ordinateur et l'imagerie optique.

4. Le poly-L-nucléotide selon la revendication 2 ou 3, dans lequel le marqueur est choisi parmi le groupe comprenant les ions de métal paramagnétiques, ⁷⁵Br, ¹¹C, ¹³C, ¹⁸F, ⁶⁷Ga, ¹¹¹In, ¹²⁰I, ¹²³I, ¹²⁴I, ¹²⁵I, ¹³¹I, ¹³N, ¹⁵O, ¹⁸⁶Re, ¹⁸⁸Re, ^{94m}Tc, ^{99m}Tc, ¹²⁹Xe gazeux, ⁸⁶Y et ⁹⁰Y.

5. Le poly-L-nucléotide selon l'une quelconque des revendications 1 à 4, dans lequel le poly-L-nucléotide est un poly-L-désoxyribonucléotide.

6. Le poly-L-nucléotide selon l'une quelconque des revendications 1 à 5, dans lequel le poly-L-nucléotide est un poly-L-ribonueléotide.

7. Le poly-L-nucléotide selon l'une quelconque des revendications 1 à 6, dans lequel l'imagerie in vivo est employée pour le diagnostic, le contrôle, l'évaluation et/ou la détermination du stade d'une maladie ou d'une affection, et/ou de la réponse d'un patient à un traitement médicamenteux.

8. Le poly-L-nucléotide selon la revendication 7, dans lequel la maladie ou l'affection est choisie parmi le groupe comprenant le cancer, la sclérose en plaques, l'arthrite rhumatoïde, un accident vasculaire, l'infarctus du myocarde, les maladies neurodégénératives et une inflammation.

9. Le poly-L-nucléotide selon la revendication 8, dans laquelle les cellules cancéreuses ont une surexpression d'au moins un type de récepteur.

10. Le poly-L-nucléotide selon l'une quelconque des revendications 1 à 9, dans lequel le poly-L-nucléotide est spécifique d'une cible.

11. Le poly-L-nucléotide selon la revendication 10, dans lequel la cible est une cible clé de la maladie ou l'affection.

12. Le poly-L-nucléotide selon l'une quelconque des revendications 1 à 11, dans lequel le poly-L-nucléotide a une longueur minimum, la longueur minimum étant choisie parmi le groupe comprenant 10, 15, 20, 25, 30, 35, 40, 45, 50, 55, 60, 70, 80, 90 et 100 nucléotides.

13. Le poly-L-nucléotide selon l'une quelconque des revendications 1 à 12, dans lequel imagerie in vivo est pour l'imagerie d'un organe.

14. Le poly-L-nucléotide selon la revendication 13, dans lequel le poly-L-nucléotide est spécifique d'un organe.

15. Le poly-L-nucléotide selon la revendication 14, dans lequel le poly-L-nucléotide est spécifique d'un composé clé de l'organe.

16. Le poly-L-nucléotide selon l'une quelconque des revendications 1 à 15, dans lequel le poly-L-nucléotide est un L-ARN, et l'imagerie in vivo est pour l'imagerie du rein.

17. Le poly-L-nucléotide selon l'une quelconque des revendications 1 à 16, dans lequel le poly-L-nucléotide est un L-ADN, et l'imagerie in vivo est pour l'imagerie du plasma, du coeur, des poumons et/ou du foie.

18. L'utilisation d'un kit comprenant un poly-L-nucléotide pour une imagerie in vivo, dans laquelle le poly-L-nucléotide est un Spiegelmer, dans lequel le Spiegelmer manifeste une activité de liaison à l'égard d'une cible ou d'une partie de celle-ci, et la cible est une protéine, un peptide, une petite molécule, ou un composé pharmaceutiquement actif et son métabolite.
